# EUROPEAN PATENT SPECIFICATION

(11) **EP 4 003 412 B1**
(45) Date of publication and mention of the grant of the patent: **24.06.2026**
(21) Application number: 20751252.6
(22) Date of filing: 28.07.2020
(51) Int. Cl.: A61K 39/12, C07K 14/00

(54) **AFRICAN SWINE FEVER VACCINE**
IMPFSTOFF GEGEN DAS AFRIKANISCHE SCHWEINEFIEBER
VACCIN CONTRE LA PESTE PORCINE AFRICAINE

(30) Priority: 29.07.2019 GB 201910794
(43) Date of publication of application: 01.06.2022
(73) Proprietor: The Pirbright Institute, Pirbright Woking Surrey GU24 0NF (GB)
(72) Inventor: DIXON, Linda, Woking Surrey GU24 0NF (GB); NETHERTON, Chris, Woking Surrey GU24 0NF (GB); TAYLOR, Geraldine, Woking Surrey GU24 0NF (GB); CHAPMAN, Dave, Woking Surrey GU24 0NF (GB)
(74) Representative: D Young & Co LLP
(86) International application number: PCT/GB2020/051808
(87) International publication number: WO 2021/019232

(56) References cited:
- EP-A1- 2 154 146
- WO-A2-2017/096341
- NEILAN J G ET AL: "Neutralizing antibodies to African swine fever virus proteins p30, p54, and p72 are not sufficient for antibody-mediated protection", VIROLOGY, ELSEVIER, AMSTERDAM, NL, vol. 319, no. 2, 20 February 2004 (2004-02-20), pages 337 - 342, XP004491680, ISSN: 0042-6822, DOI: 10.1016/J.VIROL.2003.11.011

## Description

### FIELD OF THE INVENTION

The present invention relates to a vaccine for the treatment and/or prevention of African swine fever.

### BACKGROUND TO THE INVENTION

African swine fever (ASF) is a devastating haemorrhagic disease of domestic pigs caused by a double-stranded DNA virus, African swine fever virus (ASFV). ASFV is the only member of the *Asfarviridae* family which replicates predominantly in the cytoplasm of cells. Virulent strains of ASFV can kill domestic pigs within about 5-14 days of infection with a mortality rate approaching 100%.

There is currently no treatment for ASF. Prevention in countries outside Africa has been attempted on a national basis by restrictions on incoming pigs and pork products, compulsory boiling of waste animal products under licence before feeding to pigs and the application of a slaughter policy when the disease is diagnosed. Prevention in Africa of spread from wildlife is based on measures to keep warthogs and materials contaminated by warthogs away from the herd. Spread in domestic pig populations is based on good biosecurity measures and implementation of quarantine and slaughter of pigs on affected farms.

To date, no effective attenuated or inactivated virus vaccines have been developed (see http://www.thepigsite.com/pighealth/article/441/african-swine-fever-asf).

Experimental protection against lethal challenge is possible by using a low virulent ASFV strain OURT88/3 that was recovered from a soft tick. OURT88/3 is a non-pathogenic isolate of ASFV from Portugal. Previous infection with ASFV OURT88/3 has been shown to confer protection against challenge with related virulent viruses (Boinas et al (2004) J Gen Virol 85:2177-2187; Oura et al (2005) J. Gen. Virol. 86:2445-2450). In addition some gene-deleted viruses are also effective in inducing protection against challenge (eg BeninΔMGF and BeninΔDP148R).

Although these viruses have been shown to induce a protective immune response in certain animals, this effect does not appear to be universal. Immunisation appears to be ineffective in protecting some pigs from subsequent challenge. It can is also be associated with the induction of adverse clinical responses, such as fever or joint swelling, in some pigs.

The ability of subunit compositions comprising ASFV polypeptides to induce protection against ASFV has also been investigated. However, these studies have determined that the ability of an ASFV polypeptide to induce an immunogenic response does not necessarily predict an ability to induce protection to ASFV challenge (see Neilan et al.; Virology; 319(2); 337-342; 2004). Further, ASFV polypeptides may only induce protection when used in specific combinations. For example, Neilan *et al.* (as above) reported that a combination of p30, p54, p72 and p22 was not sufficient to induce protection. In contrast, Argilaguet et al. (Antiviral Res; 98(1); 61-65; 2013) reported that a combination of p30, p54 and EP402R gave partial protection against ASFV in DNA vaccination experiments.

WO 2017/096341 A2 discloses a vaccine comprising an immunologically effective amount of a novel live-vectored multivalent vaccine formulation that affords immunization to multiple antigens of a pathogen that is relatively impervious to vaccine development by providing multiple virus-expressed antigens and a pharmaceutically acceptable carrier and/or an adjuvant and methods of using said vaccine.

EP 2 154 146 A1 discloses the use of the hemagglutinin (HA) of African swine fever virus (ASFV) as an adjuvant to enhance the immune response against an antigen in a subject.

There is thus a need for alternative measures to control ASFV infection and prevent spread of the disease.

### SUMMARY OF ASPECTS OF THE INVENTION

The present inventors have determined a minimal combination of three ASFV polypeptides that are capable of inducing repeatable protection against ASFV when administered prior to ASFV challenge. The inventors have determined that protection is not induced when any one of the three ASFV polypeptides is absent. Further, the inventors have demonstrated that this minimal combination of three ASFV polypeptides may be combined with additional ASFV polypeptides to provide improved vaccine compositions for inducing reliable protection against ASFV challenge and treatment and/or prevention of ASF.

Thus, in one aspect, the present invention provides an African swine fever virus (ASFV) subunit vaccine comprising:
(i) one or more recombinant polynucleotides which encode polypeptides shown as:
   (a) SEQ ID NO: 1 or a variant with at least 95% sequence identity thereto,
   (b) SEQ ID NO: 2 or a variant with at least 95% sequence identity thereto, and
   (c) SEQ ID NO: 3 or a variant with at least 95% sequence identity thereto; wherein the total number of different ASFV polypeptides encoded by the one or more recombinant polynucleotides is 10 or fewer; or
(ii) recombinant polypeptides shown as
   (a) SEQ ID NO: 1 or a variant with at least 95% sequence identity thereto,
   (b) SEQ ID NO: 2 or a variant with at least 95% sequence identity thereto, and
   (c) SEQ ID NO: 3 or a variant with at least 95% sequence identity thereto; wherein vaccine comprises 10 or fewer different ASFV polypeptides.

The term subunit vaccine is used herein to refer to a vaccine which comprises individual polypeptides or polynucleotides encoding said polypeptides; in contrast to vaccines which comprise whole virus particles (such a live, attenuated virus and dead virus).

The number of different ASFV polypeptides encoded by the one or more recombinant polynucleotides may be 9 or fewer, 8 or fewer, 7 or fewer, or 6 or fewer polypeptides ASFV polypeptides; or the vaccine may comprise 9 or fewer, 8 or fewer, 7 or fewer, or 6 or fewer.

The vaccine may comprise: (i) one or more further recombinant polynucleotides encoding an ASFV polypeptide selected from SEQ ID NO: 4-8, or a variant with at least 95% sequence identity to one of SEQ ID NO: 4-8; or (ii) one or more further polypeptides selected from SEQ ID NO: 4-8; or a variant with at least 95% sequence identity to one of SEQ ID NO: 4-8.

The vaccine according may comprise: a) a recombinant polynucleotide(s) which encodes polypeptides comprising SEQ ID NO: 1-8 or a variant with at least 95% sequence identity to SEQ ID NO: 1-8; or recombinant polypeptides comprising SEQ ID NO: 1-8 or variants with at least 95% sequence identity to SEQ ID NO: 1-8; (b) a recombinant polynucleotide(s) which encodes polypeptides comprising SEQ ID NO: 1-3 and 6-8 or variants with at least 95% sequence identity to SEQ ID NO: 1-3 and 6-8; or recombinant polypeptides comprising SEQ ID NO: 1-3 and 6-8 or variants with at least 95% sequence identity to SEQ ID NO: 1-3 and 6-8; or (c) a recombinant polynucleotide(s) which encode polypeptides comprising SEQ ID NO: 1-5 or variants with at least 95% sequence identity to SEQ ID NO: 1-5; or recombinant polypeptides comprising SEQ ID NO: 1-5 or variants with at least 95% sequence identity to SEQ ID NO: 1-5.

The recombinant polynucleotides may comprise SEQ ID NO: 9, 10 and 11 or a variant thereof with at least 95% sequence identity.

The further polynucleotides may comprise one or more of SEQ ID NO: 12-16 or a variant thereof with at least 95% sequence identity.

The polynucleotide may be present in a vector. The vector may be selected from an adenovirus, a modified vaccinia Ankara vector and a pseudorabies virus vector.

Each of the recombinant polynucleotides may be provided in the same vector.

In a further aspect the present invention provides a vaccine according to the invention for use in treating and/or preventing African swine fever in a subject.

The subject may be a swine subject. Suitably, the subject may be a domestic pig.

The vaccine may be administered according to a prime-boost procedure. For example, the priming composition may comprise one or more adenovirus vectors and the boosting composition may comprise one or more modified vaccinia Ankara vectors.

The vaccine may be administered by oral, intravenous, intramuscular, subcutaneous, intranasal or intradermal administration.

### DESCRIPTION OF THE FIGURES

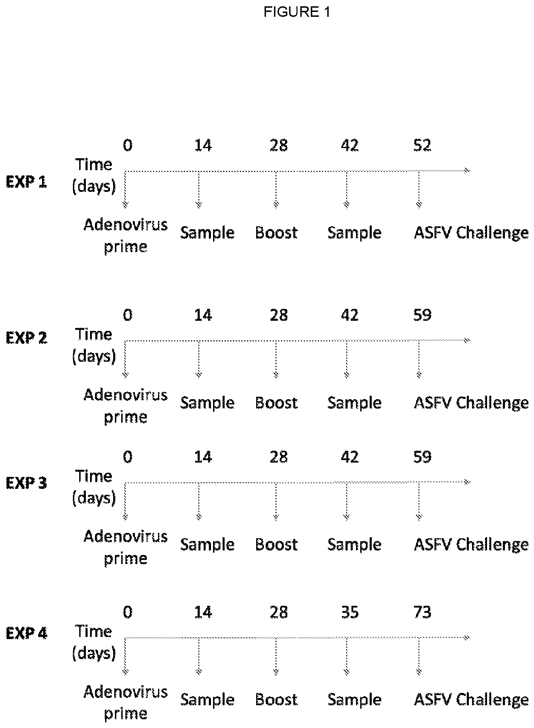

**Figure 1** - Scheme of Adenovirus-MVA prime/boost protocol
**Figure 2** - Representative amino acid sequences for ASFV polypeptides for use in the present invention (SEQ ID NO: 1-8 and 17)
**Figure 3** - Representative nucleic acid sequences encoding ASFV polypeptides for use in the present invention (SEQ ID NO: 9-16)

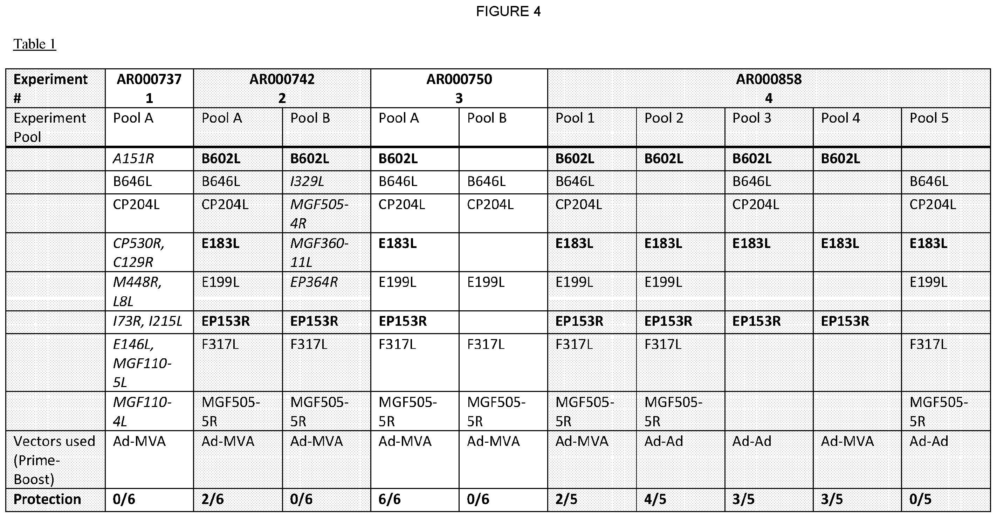

**Figure 4** - Table 1

### DETAILED DESCRIPTION OF THE INVENTION

### AFRICAN SWINE FEVER VIRUS (ASFV)

The present inventors have determined a minimal combination of three ASFV polypeptides that are capable of inducing repeatable protection against ASFV.

African swine fever virus (ASFV) is the causative agent of African swine fever (ASF). The virus causes a haemorrhagic fever with high mortality rates in pigs, but persistently infects its natural hosts, warthogs, bushpigs with no disease signs. It also infects soft ticks of the *Ornithodoros* genus, which are thought to be used as a vector.

ASFV replicates in the cytoplasm of infected cells, and is the only member of the *Asfarviridae* family to do so. ASFV is endemic to sub-Saharan Africa and exists in the wild through a cycle of infection between ticks and wild pigs, bushpigs and warthogs. ASFV was first described after European settlers brought pigs into areas endemic with ASFV and, as such, is an example of an 'emerging infection'.

ASFV is a large, icosahedral, double-stranded DNA virus with a linear genome containing at least 150 genes. The number of genes differs slightly between different isolates of the virus. ASFV has similarities to the other large DNA viruses, e.g., poxvirus, iridovirus and mimivirus. In common with other viral haemorrhagic fevers, the main target cells for replication are those of monocyte, macrophage lineage.

Based on sequence variation in the C-terminal region of the B646L gene encoding the major capsid protein p72, 24 ASFV genotypes (I-XXIX) have been identified. All ASFV p72 genotypes have been circulating in eastern and southern Africa. Genotype I is been circulating in Sardinia and western Africa. Genotype II is circulating in Europe, China, Vietnam and Camboida. Genotype VIII is confined to four East African countries.

Examples of strains from some of the genotypes are given below. Strains shown in bold were used in the identity analysis shown in Table 2.
Genotype I : **OURT88/3;** Brazil/79; Lisbon/60; BA715; **ASFV-Pret; Benin 97/1;** IC/1/96; IC/576; CAM/82; Madrid/62; Malta/78; ZAR85; Katange63; Togo; Dakar59; OURT88/1; BEN/1/97; Dom_Rep; VAL/76; IC/2/96; Awoshie/99; NIG/1/99; NIG/1/98; ANG/70; BEL/85; SPEC120; Lisbon/57; **ASFV-Warm;** GHA/1/00; GAM/1/00; Ghana; HOL/86; NAM/1/80; NUR/90/1; CAM/4/85; **ASFV-Teng;** Tengani; **ASFV-E75.**
Genotype II: **Georgia 2007/1,** Belgium 2018/1, China/2018/AnhuiXCGQ
Genotype III: BOT 1/99
Genotype IV: **ASFV-Warthog;** RSA/1/99/W
Genotype VI: MOZ 94/1
Genotype VII: VICT/90/1; **ASFV-Mku;** RSA/1/98
Genotype VIII: NDA/1/90; KAL88/1; ZAM/2/84; JON89/13; KAV89/1; DEZda; **Malawi LIL 20/1**
Genotype IX: UGA/1/95; **Ken06.Bus**
Genotype X: BUR/1/84; BUR/2/84; BUR/90/1; UGA/3/95; TAN/Kwh12; Hindell; **ASFV-Ken;** Virulent Uganda 65; **Ken05/Tk1.**

The present inventors have determined that the composition comprising a minimal combination of ASFV polypeptides SEQ ID NO: 1-3 are capable of inducing protection against ASFV challenge.

**Table 2**

| Gene Name | Alternative Gene Names | Amino acid SEQ ID NO: | cDNA SEQ ID NO: |
|---|---|---|---|
| B602L | ASFV chaperone | 1 | 9 |
| E183L | p54/j13L | 2 | 10 |
| EP153R | ASFV lectin | 3 | 11 |
| B646L | p72 | 4 | 12 |
| CP204L | CP196L / p30 | 5 | 13 |
| E199L | | 6 | 14 |
| F317L | | 7 | 15 |
| MGF505-5R | | 8 | 16 |

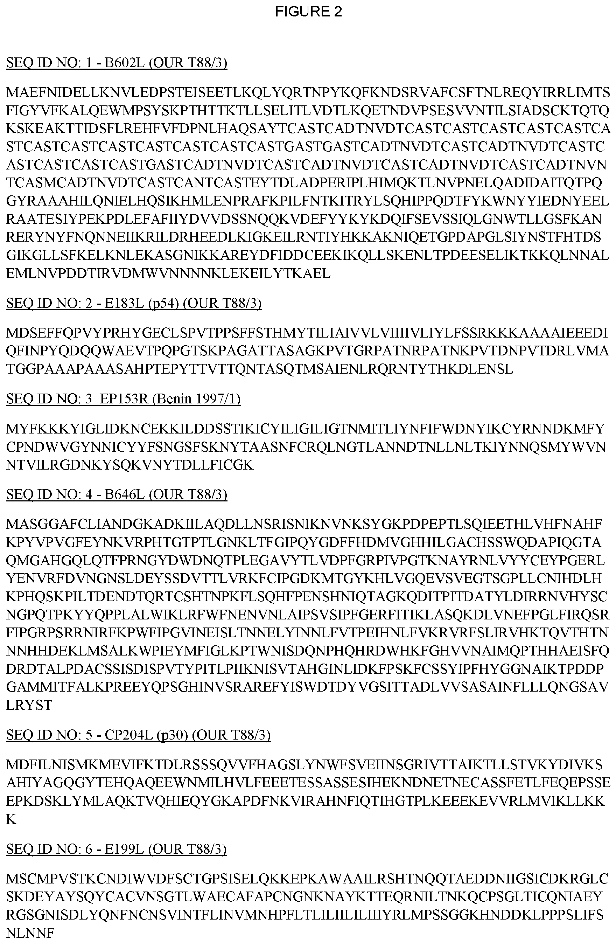

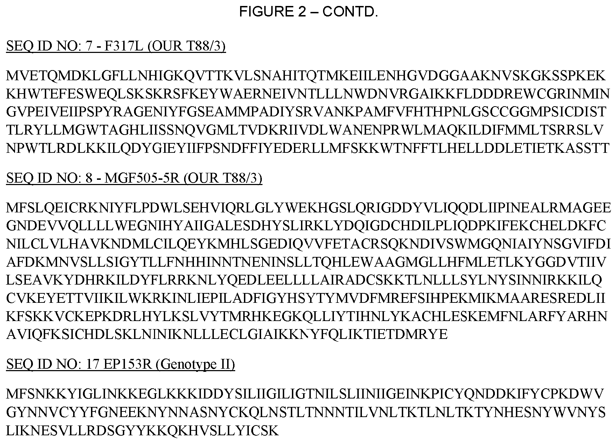

Representative amino acid sequences of polypeptides in Table 2 are shown in Figure 2 (SEQ ID NO: 1-8 and 17).

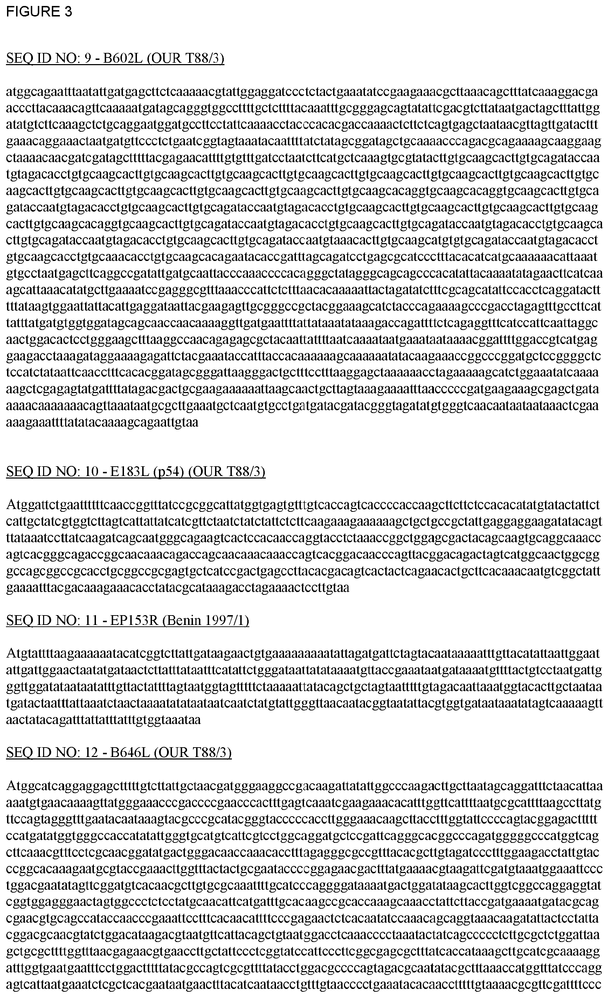

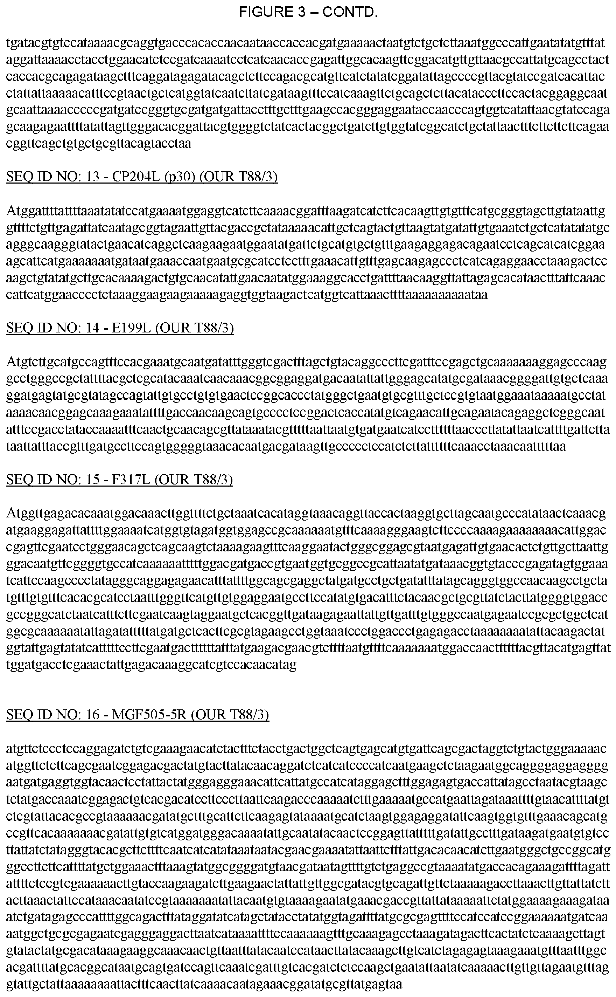

Representative nucleic acid sequences encoding the polypeptides in Table 2 are shown as SEQ ID NO: 9-16 in Figure 3.

### POLYPEPTIDE

The term "polypeptide" is used in the normal sense to mean a series of residues, typically L-amino acids, connected one to the other typically by peptide bonds between the α-amino and carboxyl groups of adjacent amino acids. The term is synonymous with "protein".

The term "recombinant polypeptide" is used to mean that the polypeptide is isolated from its natural environment, for example, extracted from or produced outside of an ASFV. In particular, the recombinant polypeptide is not provided in the context of an ASFV and is expressed from a recombinant nucleic acid, i.e. DNA or RNA that is created artificially.

The vaccine of the present invention may comprise a combination of ASFV polypeptides, as defined herein.

The present vaccine may comprise recombinant polypeptides shown as SEQ ID NO: 1, 2 and 3; or a variant with at least 95% sequence identity to one of SEQ ID NO: 1, 2 and 3.

In any embodiment of the present invention, the polypeptide of SEQ ID NO: 3; or a variant with at least 95% sequence identity to one of SEQ ID NO: 3 may be substituted with SEQ ID NO: 17; or a variant with at least 95% sequence identity to one of SEQ ID NO: 17.

SEQ ID NO: 17 is a representative EP153R sequence from a Genotype II strain.

Suitably, the variant may comprise an amino acid sequence which is at least 95, 98 or 99% identical to one of SEQ ID NO: 1, 2 or 3.

Suitably, the variant may comprise an amino acid sequence which is at least 95, 98 or 99% identical to SEQ ID NO: 1.

Suitably, the variant may comprise an amino acid sequence which is at least 95, 98 or 99% identical to SEQ ID NO: 2.

Suitably, the variant may comprise an amino acid sequence which is at least 95, 98 or 99% identical to SEQ ID NO: 3.

The present vaccine may comprise recombinant polypeptides shown as SEQ ID NO: 1, 2 and 3; or a variant with at least 95% sequence identity to one of SEQ ID NO: 1, 2 and/or 3. In one embodiment, the vaccine may consist of recombinant polypeptides shown as SEQ ID NO: 1, 2 and 3; or a variant with at least 95% sequence identity to one of SEQ ID NO: 1, 2 and/or 3.

In one embodiment, the recombinant polypeptides may consist of amino acid sequences shown as SEQ ID NO: 1, 2 and 3 or a variant thereof which shares at least 95% sequence identity

The vaccine may further comprise one or more polypeptides selected from SEQ ID NO: 4-8; or a variant with at least 95% sequence identity to one of SEQ ID NO: 4-8.

Suitably, the variant may comprise an amino acid sequence which is at least 95, 98 or 99% identical to one of SEQ ID NO: 4-8.

Suitably, the variant may comprise an amino acid sequence which is at least 95, 98 or 99% identical to SEQ ID NO: 4.

Suitably, the variant may comprise an amino acid sequence which is at least 95, 98 or 99% identical to SEQ ID NO: 5.

Suitably, the variant may comprise an amino acid sequence which is at least 95, 98 or 99% identical to SEQ ID NO: 6.

Suitably, the variant may comprise an amino acid sequence which is at least 95, 98 or 99% identical to SEQ ID NO: 7.

Suitably, the variant may comprise an amino acid sequence which is at least 95, 98 or 99% identical to SEQ ID NO: 8.

The vaccine may further comprise one or more polypeptides selected from SEQ ID NO: 4-8 or a variant with at least 95% sequence identity to one of SEQ ID NO: 4-8.

The vaccine may comprise at least 2, at least 3, at least 4 or at least 5 polypeptides selected from SEQ ID NO: 4-8; or a variant with at least 95% sequence identity to one of SEQ ID NO: 4-8.

The vaccine may comprise 2, 3, 4 or 5 polypeptides selected from SEQ ID NO: 4-8; or a variant with at least 95% sequence identity to one of SEQ ID NO: 4-8.

Suitably, the vaccine may comprise recombinant polypeptides comprising SEQ ID NO: 1-8; or variants with at least 95% sequence identity to SEQ ID NO: 1-8.

Suitably, the vaccine may comprise recombinant polypeptides comprising SEQ ID NO: 1-3 and 6-8; or variants with at least 95% sequence identity to SEQ ID NO: 1-3 and 6-8.

Suitably, the vaccine may comprise recombinant polypeptides comprising SEQ ID NO: 1-5; or variants with at least 95% sequence identity to SEQ ID NO: 1-5.

### VARIANT

Although a variant can be considered in terms of similarity (i.e. amino acid residues having similar chemical properties/functions), in the context of the present invention it is preferred to express a variant in terms of sequence identity.

Sequence comparisons can be conducted by eye, or more usually, with the aid of readily available sequence comparison programs. These publicly and commercially available computer programs can calculate sequence identity between two or more sequences.

Sequence identity may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the ^{a}lignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % sequence identity therefore firstly requires the production of an optimal alignment, taking into consideration gap penalties. A suitable computer program for carrying out such an alignment is the GCG Wisconsin Bestfit package (University of Wisconsin, U.S.A; Devereux et al., 1984, Nucleic Acids Research 12:387). Examples of other software than can perform sequence comparisons include, but are not limited to, the BLAST package (see Ausubel *et al.,* 1999 *ibid* - Chapter 18), FASTA (Atschul et al., 1990, J. Mol. Biol., 403-410) and the GENEWORKS suite of comparison tools. Both BLAST and FASTA are available for offline and online searching (see Ausubel *et al.,* 1999 *ibid,* pages 7-58 to 7-60). However it is preferred to use the GCG Bestfit program.

In one embodiment, the sequence identity is determined across the entirety of the sequence selected from SEQ ID NO: 1-8 or SEQ ID NO: 9-16. In one embodiment, the sequence identity is determined across the entirety of the candidate sequence being compared to a sequence selected from SEQ ID NO: 1-8 or SEQ ID NO: 9-16.

Although the final sequence identity can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % sequence identity. The software typically does this as part of the sequence comparison and generates a numerical result.

The term "variant" according to the present invention includes any substitution of, variation of, modification of, replacement of, deletion of or addition of one (or more) amino acids from or to the sequence providing the resultant amino acid sequence retains substantially the same activity as the unmodified sequence.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

With respect to function, the variant should be capable of inducing an immune response. By way of example, the induction of an immune response may be determined by the demonstration of a recall response in peripheral immune cells or splenocytes isolated from a subject previously immunised with the polypeptide or immunogenic fragment thereof. For instance, a recall response may be demonstrated by interferon production (e.g. IFNγ) and/or a proliferative response following an *in vitro* challenge with an antigen or polypeptide previous used to immunise a subject. Preferably, the variant should be capable of inducing a protective immune response in a subject, against subsequent challenge with ASFV when administered in combination with a polypeptide selected from SEQ ID NO: 1-3 or a variant as described herein.

### POLYNUCLEOTIDE

The polynucleotide may be any suitable type of polynucleotide, such as a synthetic RNA/DNA sequence, a cDNA sequence or a partial genomic DNA sequence.

As used herein, the terms and nucleic acid sequence and polynucleotide are intended to be synonymous with each other. "Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

The term "recombinant polynucleotide" is used to mean that the polynucleotide is isolated from its natural environment, for example, extracted from or produced outside of an ASFV. In particular, the recombinant polynucleotide is not provided in the context of an ASFV and is provided as a polynucleotide, i.e. DNA or RNA that is created artificially.

It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides described here to reflect the codon usage of any particular host organism in which the polypeptides are to be expressed.

The term "variant" is used to mean a naturally occurring nucleic acid sequence which differs from a subject sequence. The variant may have at least 95% sequence identity with the subject sequence.

The present vaccine may comprise one or more polynucleotides which encode a combination of recombinant polypeptides according to the vaccine of the present invention.

Illustrative polynucleotides encoding each of polypeptides SEQ ID NO: 1-8 are shown in Table 2.

The present vaccine may comprise one or more polynucleotides which encode recombinant polypeptides shown as SEQ ID NO: 1, 2 and 3; or a variant with at least 95% sequence identity to one of SEQ ID NO: 1, 2 and 3.

The present vaccine may comprise one or more polynucleotides which encode recombinant polypeptides shown as SEQ ID NO: 1, 2 and 3; or a variant with at least 95% sequence identity to one of SEQ ID NO: 1, 2 and/or 3. In one embodiment, the vaccine may consist of one or more polynucleotides which encode recombinant polypeptides shown as SEQ ID NO: 1, 2 and 3; or a variant with at least 95% sequence identity to one of SEQ ID NO: 1, 2 and/or 3.

The vaccine may further comprise one or more polynucleotides which encode one or more polypeptides selected from SEQ ID NO: 4-8 ; or a variant with at least 95% sequence identity to one of SEQ ID NO: 4-8.

The vaccine may further comprise one or more polynucleotides which encode one or more polypeptides selected from SEQ ID NO: 4-8 or a variant with at least 95% sequence identity to one of SEQ ID NO: 4-8.

The vaccine may comprise one or more polynucleotides which encode at least 2, at least 3, at least 4 or at least 5 polypeptides selected from SEQ ID NO: 4-; or a variant with at least 95% sequence identity to one of SEQ ID NO: 4-8.

The vaccine may comprise one or more polynucleotides which encode 2, 3, 4 or 5 polypeptides selected from SEQ ID NO: 4-8; or a variant with at least 95% sequence identity to one of SEQ ID NO: 4-8.

Suitably, the vaccine may comprise one or more polynucleotides which encode recombinant polypeptides comprising SEQ ID NO: 1-8; or variants with at least 95% sequence identity to SEQ ID NO: 1-8.

Suitably, the vaccine may comprise one or more polynucleotides which encode recombinant polypeptides comprising SEQ ID NO: 1-3 and 6-8 ; or variants with at least 95% sequence identity to SEQ ID NO: 1-3 and 6-8.

Suitably, the vaccine may comprise one or more polynucleotides which encode recombinant polypeptides comprising SEQ ID NO: 1-5; or variants with at least 95% sequence identity to SEQ ID NO: 1-5.

A polynucleotide encoding the polypeptide shown as SEQ ID NO: 1-3 or a variant thereof may comprise SEQ ID NO: 9-11, respectively; or a variant thereof which shares at least 95% sequence identity to one of SEQ ID NO: 9-11.

A polynucleotide encoding the polypeptide shown as SEQ ID NO: 1 or a variant thereof may comprise SEQ ID NO: 9; or a variant thereof which is at least 95, 98 or 99% identical to SEQ ID NO: 9.

A polynucleotide encoding the polypeptide shown as SEQ ID NO: 2 or a variant thereof may comprise SEQ ID NO: 10; or a variant thereof which is at least 95, 98 or 99% identical to SEQ ID NO: 10.

A polynucleotide encoding the polypeptide shown as SEQ ID NO: 3 or a variant thereof may comprise SEQ ID NO: 11; or a variant thereof which is at least 95, 98 or 99% identical to SEQ ID NO: 11.

A polynucleotide encoding the polypeptide shown as SEQ ID NO: 4-8 or a variant thereof may comprise SEQ ID NO: 12-16, respectively; or a variant thereof which shares at least 95% sequence identity to one of SEQ ID NO: 12-16.

A polynucleotide encoding the polypeptide shown as SEQ ID NO: 4 or a variant thereof may comprise SEQ ID NO: 12; or a variant thereof which is at least 95, 98 or 99% identical to SEQ ID NO: 12.

A polynucleotide encoding the polypeptide shown as SEQ ID NO: 5 or a variant thereof may comprise SEQ ID NO: 13; or a variant thereof which is at least 95, 98 or 99% identical to SEQ ID NO: 13.

A polynucleotide encoding the polypeptide shown as SEQ ID NO: 6 or a variant thereof may comprise SEQ ID NO: 14; or a variant thereof which is at least 95, 98 or 99% identical to SEQ ID NO: 14.

A polynucleotide encoding the polypeptide shown as SEQ ID NO: 7 or a variant thereof may comprise SEQ ID NO: 15; or a variant thereof which is at least 95, 98 or 99% identical to SEQ ID NO: 15.

A polynucleotide encoding the polypeptide shown as SEQ ID NO: 8 or a variant thereof may comprise SEQ ID NO: 16; or a variant thereof which is at least 95, 98 or 99% identical to SEQ ID NO: 16.

The vaccine may further comprise one or more polypeptides selected from SEQ ID NO: 4-8 or a variant with at least 95% sequence identity to one of SEQ ID NO: 4-8.

Suitably, the vaccine may comprise one or more polynucleotides comprising SEQ ID NO: 9-16 or variants with at least 95% sequence identity to SEQ ID NO: 9-16.

Suitably, the vaccine may comprise one or more polynucleotides comprising SEQ ID NO: 9-11 and 14-16; or variants with at least 95% sequence identity to SEQ ID NO: 9-11 and 14-16.

Suitably, the vaccine may comprise one or more polynucleotides comprising SEQ ID NO: 9-13; or variants with at least 95% sequence identity to SEQ ID NO: 9-13.

### COMBINATION

The present invention is based, at least in part, on the inventors' determination of a minimal combination of polypeptides, which is capable of inducing protection against ASFV.

It is to be understood that references to polypeptides or combinations of polypeptides provided herein are intended to apply equally to corresponding polynucleotides or combinations of polynucleotides which encode said polypeptides.

The total number of different ASFV polypeptides (or encoded by the one or more recombinant polynucleotides) in the present vaccine is 10 or fewer.

Suitably, the number of different ASFV polypeptides is 9 or fewer, 8 or fewer, 7 or fewer, or 6 or fewer.

Suitably, the number of different ASFV polypeptides is 3, 4, 5, 6, 7 or 8.

Suitably, the number of different ASFV polypeptides is 3.

Suitably, the number of different ASFV polypeptides is 5.

Suitably, the number of different ASFV polypeptides is 6.

Suitably, the number of different ASFV polypeptides is 8.

### VECTOR

As used herein, a "vector" may be any agent capable of delivering or maintaining nucleic acid in a host cell, and includes viral, bacterial and eukaryotic vectors, plasmids, naked nucleic acids, nucleic acids complexed with polypeptide or other molecules and nucleic acids immobilised onto solid phase particles.

Polynucleotides in accordance with the present invention may be delivered by viral or non-viral techniques.

Non-infectious delivery systems include but are not limited to DNA transfection methods. Here, transfection includes a process using a non-infectious vector to deliver nucleic acid of the invention to a target cell.

Typical transfection methods include electroporation, nucleic acid biolistics, lipid-mediated transfection, compacted nucleic acid-mediated transfection, liposomes, immunoliposomes, lipofectin, cationic agent-mediated, cationic facial amphiphiles (CFAs), multivalent cations such as spermine, cationic lipids or polylysine, 1, 2,-bis (oleoyloxy)-3-(trimethylammonio) propane (DOTAP)-cholesterol complexes and combinations thereof.

Non-viral delivery systems may also include, but are not limited to, bacterial delivery systems. Bacteria have previously been used as anticancer agents and as delivery agents for anticancer drugs.

Cell adhesion molecules are a large group of molecules involved in a variety of cell-to-cell and cell-to-extra-cellular matrix (ECM) interactions and are exploited by a number of pathogenic micro-organisms as receptors for cell entry. These molecules may be used for the targeting and uptake of both gene and drug delivery systems.

A gene gun delivery system may also be used for the delivery of DNA.

Viral delivery systems include but are not limited to adenovirus vectors, modified vaccinia Ankara vectors, adeno-associated viral (AAV) vectors, herpes viral vectors such as pseudorabies virus vector, retroviral vectors, lentiviral vectors or baculoviral vectors, venezuelan equine encephalitis virus (VEE), poxviruses such as: canarypox virus, entomopox virus, penguine alphavirus, and alphavirus based DNA vectors.

In one embodiment the vector may be an adenovirus vector.

The adenovirus is a double-stranded, linear DNA virus that does not replicate through an RNA intermediate. There are over 50 different human serotypes of adenovirus divided into 6 subgroups based on their genetic sequence.

Adenoviruses are double-stranded DNA non-enveloped viruses that are capable of in vivo, ex vivo and in vitro transduction of a broad range of cell types of human and non-human origin. These cells include respiratory airway epithelial cells, hepatocytes, muscle cells, cardiac myocytes, synoviocytes, primary mammary epithelial cells and post-mitotically terminally differentiated cells such as neurons. Adenoviral vectors are also capable of transducing non-dividing cells.

Adenoviruses have been used as vectors for gene therapy and for expression of heterologous genes. The large (36 kb) genome can accommodate up to 8 kb of foreign insert DNA and is able to replicate efficiently in complementing cell lines to produce very high titres of up to 1012 transducing units per ml. Adenovirus is thus one of the best systems to study the expression of genes in primary non-replicative cells. The expression of viral or foreign genes from the adenovirus genome does not require a replicating cell. Adenoviral vectors enter cells by receptor mediated endocytosis. Once inside the cell, adenovirus vectors rarely integrate into the host chromosome. Instead, they function episomally (independently from the host genome) as a linear genome in the host nucleus.

In one embodiment, the vector may be a modified vaccinia Ankara (MVA) vector. The MVA virus is related to Vaccinia virus, a member of the genera Orthopoxvirus in the family of Poxviridae. The MVA virus has been generated by 516 serial passages on chicken embryo fibroblasts of the Chorioallantois Vaccinia Ankara (CVA) virus. In the course of the attenuation process by repeated passaging to chicken derived material as production substrate, the MVA virus has lost approximately 15% of the genomic DNA at multiple sites (Mayr and Munz 1964 in Zentralbl Bakteriol Orig 195, 24-35; Meyer et al. 1991 in J Gen Virol 72 ( Pt 5), 1031-1038). The MVA virus has been analysed to determine alterations in the genome relative to the wild-type CVA strain. Six major deletions of genomic DNA (deletion I, II, III, IV, V, and VI), totalling 31.000 base pairs, have been identified (Meyer, et al. 1991 in J Gen Virol 72 (Pt 5), 1031-1038). MVA does not replicate in human and non-human primate cells.

Alternatives to vaccinia vectors include avipox vectors such as fowlpox or canarypox known as ALVAC and strains derived therefrom which can infect and express recombinant proteins in human cells but are unable to replicate.

Examples of other vectors include *ex vivo* delivery systems, which include but are not limited to DNA transfection methods such as electroporation, DNA biolistics, lipid-mediated transfection and compacted DNA-mediated transfection.

The vector may be a plasmid DNA vector. As used herein, "plasmid" refers to discrete elements that are used to introduce heterologous DNA into cells for either expression or replication thereof.

In one embodiment, the vector may comprise more than one nucleic acid sequences, each of which encodes a different polypeptide as described for use in the present vaccine.

Suitably, one or more vectors may comprise multiple nucleic acid sequences which between them encode a combination of polypeptide as described for use in the present vaccine

In one embodiment, the vector may comprise more than one nucleic acid sequence, such that the vector comprises nucleic acid sequences which between them encode a combination of polypeptide as described for use in the present vaccine.

In order for the more than one nucleic acid sequences to be expressed, there may be two or more transcription units within the vector, one for each nucleic acid sequence. In one embodiment, an internal ribosome entry site (IRES) is used to initiate translation of the second (and subsequent) coding sequence(s) in a polycistronic message (Adam et al 1991 J.Virol. 65, 4985).

Insertion of IRES elements into retroviral vectors is compatible with the retroviral replication cycle and allows expression of multiple coding regions from a single promoter (Adam et al (as above); Koo et al (1992) Virology 186:669-675; Chen et al 1993 J. Virol 67:2142-2148). IRES elements were first found in the non-translated 5' ends of picornaviruses where they promote cap-independent translation of viral proteins (Jang et al (1990) Enzyme 44: 292-309). When located between open reading frames in an RNA, IRES elements allow efficient translation of the downstream open reading frame by promoting entry of the ribosome at the IRES element followed by downstream initiation of translation.

A review on IRES is presented by Mountford and Smith (TIG May 1995 vol 11, No 5:179-184). A number of different IRES sequences are known including those from encephalomyocarditis virus (EMCV) (Ghattas, I.R., et al., Mol. Cell. Biol., 11:5848-5859 (1991); BiP protein [Macejak and Sarnow, Nature 353:91 (1991)]; the Antennapedia gene of Drosophila (exons d and e) [Oh, et al., Genes & Development, 6:1643-1653 (1992)] as well as those in polio virus (PV) [Pelletier and Sonenberg, Nature 334: 320-325 (1988); see also Mountford and Smith, TIG 11, 179-184 (1985)].

The term "IRES" includes any sequence or combination of sequences which work as or improve the function of an IRES.

The IRES(s) may be of viral origin (such as EMCV IRES, PV IRES, or FMDV 2A-like sequences) or cellular origin (such as FGF2 IRES, NRF IRES, Notch 2 IRES or EIF4 IRES).

In order for the IRES to be capable of initiating translation of each polynucleotide it should be located between or prior to the polynucleotides in the vector genome.

The polynucleotide may encode a polypeptide which comprises two or more immunogenic polypeptides for use in the present vaccine, wherein each polypeptide is joined by a cleavage site. The cleavage site may be self-cleaving, such that when the polypeptide is produced, it is immediately cleaved into the individual immunogenic polypeptides without the need for any external cleavage activity.

Various self-cleaving sites are known, including the Foot-and-Mouth disease virus (FMDV) 2a self-cleaving peptide, which has the sequence shown:
SEQ ID NO:19
   RAEGRGSLLTCGDVEENPGP.
   or
SEQ ID NO: 18
   QCTNYALLKLAGDVESNPGP

A 'self-cleaving peptide' refers to a peptide which functions such that when the nascent polypeptide comprising two or more polypeptides for use in the present vaccine and the self-cleaving peptide is produced, it is immediately "cleaved" or separated into distinct and discrete ASFV polypeptides without the need for any external cleavage activity.

In embodiments wherein the polypeptide comprises multiple ASFV polypeptides for use in the present vaccine, for example, three or more ASFV polypeptides, there may be a self-cleaving polypeptide present between each of the ASFV polypeptides. Suitably, there may be a self-cleaving polypeptide present between the first and second ASFV polypeptides, the second and third ASFV polypeptides, etc.

The self-cleaving peptide may be a 2A self-cleaving peptide from an aphtho- or a cardiovirus. The primary 2A/2B cleavage of the aptho- and cardioviruses is mediated by 2A "cleaving" at its own C-terminus. In apthoviruses, such as foot-and-mouth disease viruses (FMDV) and equine rhinitis A virus, the 2A region is a short section of about 18 amino acids, which, together with the N-terminal residue of protein 2B (a conserved proline residue) represents an autonomous element capable of mediating "cleavage" at its own C-terminus.

Expression of a nucleic acid sequence may be controlled using control sequences, which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters.

Suitable promoting sequences are strong promoters including those derived from the genomes of viruses, such as polyoma virus, adenovirus, fowlpox virus, bovine papilloma virus, avian sarcoma virus, cytomegalovirus (CMV), retrovirus and Simian Virus 40 (SV40), or from heterologous mammalian promoters, such as the actin promoter, EF1a, CAG, TK, SV40, ubiquitin, PGK or ribosomal protein promoter. Alternatively, tissue-specific promoters such as rhodopsin (Rho), rhodopsin kinase (RhoK), cone-rod homeobox containing gene (CRX), neural retina-specific leucine zipper protein (NRL), Vitelliform Macular Dystrophy 2 (VMD2), Tyrosine hydroxylase, neuronal-specific neuronal-specific enolase (NSE) promoter, astrocyte-specific glial fibrillary acidic protein (GFAP) promoter, human α1-antitrypsin (hAAT) promoter, phosphoenolpyruvate carboxykinase (PEPCK), liver fatty acid binding protein promoter, Flt-1 promoter, INF-β promoter, Mb promoter, SP-B promoter, SYN1 promoter, WASP promoter, SV40 / hAlb promoter, SV40 / CD43, SV40 / CD45, NSE / RU5' promoter, ICAM-2 promoter, GPllb promoter, GFAP promoter, Fibronectin promoter, Endoglin promoter, Elastase-1 promoter, Desmin promoter, CD68 promoter, CD14 promoter and B29 promoter may be used to drive transcription.

Transcription of a gene may be increased further by inserting an enhancer sequence into the vector. Enhancers are relatively orientation- and position-independent; however, one may employ an enhancer from a eukaryotic cell virus, such as the SV40 enhancer on the late side of the replication origin (bp 100-270) and the CMV early promoter enhancer. The enhancer may be spliced into the vector at a position 5' or 3' to the promoter, but is preferably located at a site 5' from the promoter.

The promoter can additionally include features to ensure or to increase expression in a suitable target cell. For example, the features can be conserved regions e.g. a Pribnow Box or a TATA box. The promoter may contain other sequences to affect (such as to maintain, enhance or decrease) the levels of expression of a nucleotide sequence. Suitable other sequences include the Sh1-intron or an ADH intron. Other sequences include inducible elements, such as temperature, chemical, light or stress inducible elements. Also, suitable elements to enhance transcription or translation may be present.

### VACCINE

The term 'vaccine' as used herein refers to a preparation which, when administered to a subject, induces or stimulates a protective immune response. A vaccine can render an organism immune to a particular disease, for example in the present case ASF. The vaccine of the present invention thus induces an immune response in a subject that is protective against subsequent ASFV challenge.

The present vaccine may comprise a vector, recombinant polypeptide and/or recombinant polynucleotide as described herein.

In one embodiment, the present vaccine comprises a plurality of vectors, recombinant polypeptides and/or recombinant polynucleotides as defined herein.

The term 'plurality' is used herein to mean more than one vector, recombinant polypeptide and/or recombinant polynucleotide as described herein. For example, a plurality may mean two, three, four, five, six, seven, eight, nine, or ten recombinant polypeptides and/or recombinant polynucleotides as defined herein.

The vaccine may be useful in preventing African Swine Fever.

The vaccine composition may optionally comprise a pharmaceutically acceptable carrier, diluent, excipient or adjuvant. The choice of pharmaceutical carrier, excipient or diluent can be selected with regard to the intended route of administration and standard pharmaceutical practice. The vaccine composition may comprise as (or in addition to) the carrier, excipient or diluent, any suitable binder(s), lubricant(s), suspending agent(s), coating agent(s), solubilising agent(s), and other carrier agents that may aid or increase the delivery or immunogenicity of the vaccine.

Such a formulation may, for example, be in a form suitable for oral, intravenous, intramuscular, subcutaneous, intranasal, intradermal administration or suppository routes or implanting (e.g. using slow release molecules).

The vaccine may additionally comprise an adjuvant. Examples of adjuvants include but are not limited to aluminium salts, oil emulsions and bacterial components (e.g. LPS and liposomes).

### METHODS OF PREVENTION/TREATMENT

The present invention further provides a vaccine of the present invention for use in treating and/or preventing ASF in a subject.

The term 'preventing' is intended to refer to averting, delaying, impeding or hindering the contraction of ASF. The vaccine may, for example, prevent or reduce the likelihood of an infectious ASFV entering a cell.

The term "treating" is intended to refer to reducing or alleviating at least one symptom of an existing ASF infection.

### ADMINISTRATION

The vaccine may be administered in a convenient manner such as by the oral, intravenous, intramuscular, subcutaneous, intranasal, intradermal or suppository routes or implanting (e.g. using slow release molecules).

Typically, a veterinarian or producer will determine the actual dosage which will be most suitable for an individual subject or group of subjects and it may vary, for example, with the age, weight and response of the particular subject(s). The dosage is such that it is sufficient to reduce and/or prevent disease symptoms.

Those skilled in the art will appreciate, for example, that route of delivery (e.g., oral vs intravenous vs subcutaneous vs intratumoural, etc) may impact dose amount and/or required dose amount may impact route of delivery. For example, where particularly high concentrations of an agent within a particular site or location (e.g., within a tumour) are of interest, focused delivery (e.g., in this example, intratumoural delivery) may be desired and/or useful. Other factors to be considered when optimizing routes and/or dosing schedule for a given therapeutic regimen may include, for example, the particular cancer being treated (e.g., type, stage, location, etc.), the clinical condition of a subject (e.g., age, overall health, etc.), the presence or absence of combination therapy, and other factors known to medical practitioners.

The dosage is such that it is sufficient to stabilise or improve symptoms of the disease.

The vaccine may be administered following a prime-boost regime. For example, after the first inoculation, the subject may receive a second boosting administration some time (e.g. 3 to 21, 3 to 14, 5 to 14 or 5 to 7 days) later. For example, the subject may receive a second boosting administration about 3, 5, 7, 14 or 21 days after the first inoculation.

In one embodiment, the first inoculation may be administered as one or more adenovirus vectors as described herein; and the second inoculation may be administered as one or more MVA vectors as defined herein. Typically the boosting administration is at a higher dose than the priming administration.

For all vertebrate use, the vector, immunogenic composition or vaccine may be administered parenterally, by injection, for example, either subcutaneously or intramuscularly. Additional formulations which are suitable for other modes of administration include suppositories and oral formulations. Where the immunogenic composition or vaccine is lyophilised, the lyophilised material may be reconstituted prior to administration, e.g. as a suspension.

### SUBJECT

The subject may be any animal which is susceptible to ASF infection. ASF susceptible animals include domestic pigs, warthogs, bush pigs and ticks.

The subject vaccinated according to the present invention may be a domestic pig.

The subject may be susceptible to ASF infection.

Where the vaccine is used to treat an established infection, the subject may have been diagnosed as positive for the disease and/or show one or more symptom(s) associated with the infection.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise.

The terms "comprising", "comprises" and "comprised of as used herein are synonymous with "including", "includes" or "containing", "contains", and are inclusive or open-ended and do not exclude additional, non-recited members, elements or method steps. The terms "comprising", "comprises" and "comprised of also include the term "consisting of'.

The publications discussed herein are provided solely for their disclosure prior to the filing date of the present application. Nothing herein is to be construed as an admission that such publications constitute prior art to the claims appended hereto.

The invention will now be further described by way of Examples, which are meant to serve to assist one of ordinary skill in the art in carrying out the invention and are not intended in any way to limit the scope of the invention.

### EXAMPLES

### Example 1 - Immunisation of pigs with pools of subunit ASFV polypeptides and challenge with ASFV

Various polynucleotides encoding different ASFV polypeptides were individually cloned in recombinant adenovirus and modified vaccinia Ankara (MVA) vectors. The cloned sequences were from the genotype I OURT88/3 strain except for EP153R and MGF360-11L which were from the genotype I strain Benin 1997/1.

Ten groups of pigs were immunised as shown in Table 1.

Pigs in experiments AR000737 and AR000742 were primed with 5E9 IU of adenovirus and 7.5E7 pfu of MVA. Pigs in experiments AR000750 and AR000858 were primed with 1.5 × 10¹⁰ infectious units of each recombinant adenovirus vector and boosted with 2 × 10⁸ plaque forming units of each recombinant MVA vector.

Details of the different combinations of antigens, vectors used for the priming and boosting doses and induction of protection are provided in Table 1.

B602L, E183L and EP153R are present in all of the groups in which protection was observed after challenge. Protection was determined when animals did not reach the predetermined humane endpoints of the study.

However, both B602L and EP153R were also in Pool B of Experiment 2 in which no protection was observed. E183L was in Pool 5 of Experiment 4 in which protection was observed after challenge. Therefore, the combination of B602L and EP153R alone was not sufficient for protection alone, likewise E183L alone is not sufficient for protection.

### Materials & Methods

### Materials and Methods

### African swine fever viruses and cells

The OUR T88/1 isolate was grown in primary macrophages from pig bone marrow. Virus stocks were prepared from a spleen suspension from an infected pig and titres were determined by limiting dilution using haemadsorption to detect virus infected cells and titres were calculated by the Spearman-Karber method

### Pig immunisation and challenge

Pigs were either outbred cross-bred Large White and Landrace or outbred cross-bred White, Landrace and Hampshire from a high health status farm or. Pigs were of average size 20 kg at the start of experiments.

Recombinant adenoviruses (rAds) and modified vaccinia Ankara viruses were delivered by intramuscular injection in at most 5 ml total volume at two separate sites. MVA or rAd boosts were delivered to pigs 4 weeks after the initial rAd prime. Pigs were challenged intramuscularly with 10⁴ HAD₅₀ Georgia 2007/1 virus four to eight weeks after the boost. Pigs were observed for development of clinical signs and these were scored using the scoring system used previously. Blood and tissue samples were collected to measure levels of virus replication.

Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

## Claims

1. An African swine fever virus (ASFV) subunit vaccine comprising:
(i) one or more recombinant polynucleotides which encode polypeptides shown as:
(a) SEQ ID NO: 1 or a variant with at least 95% sequence identity thereto,
(b) SEQ ID NO: 2 or a variant with at least 95% sequence identity thereto, and
(c) SEQ ID NO: 3 or a variant with at least 95% sequence identity thereto; wherein the total number of different ASFV polypeptides encoded by the one or more recombinant polynucleotides is 10 or fewer; or
(ii) recombinant polypeptides shown as
(a) SEQ ID NO: 1 or a variant with at least 95% sequence identity thereto,
(b) SEQ ID NO: 2 or a variant with at least 95% sequence identity thereto, and
(c) SEQ ID NO: 3 or a variant with at least 95% sequence identity thereto; wherein vaccine comprises 10 or fewer different ASFV polypeptides.

2. A vaccine according to claim 1, wherein the number of different ASFV polypeptides encoded by the one or more recombinant polynucleotides is 9 or fewer, 8 or fewer, 7 or fewer, or 6 or fewer; or wherein the vaccine comprises 9 or fewer, 8 or fewer, 7 or fewer, or 6 or fewer ASFV polypeptides.

3. A vaccine according to claim 1 or 2 which comprises:
(i) one or more further recombinant polynucleotides encoding an ASFV polypeptide selected from SEQ ID NO: 4-8, or a variant with at least 95% sequence identity to one of SEQ ID NO: 4-8; or
(ii) one or more further polypeptides selected from SEQ ID NO: 4-8; or a variant with at least 95% sequence identity to one of SEQ ID NO: 4-8.

4. A vaccine according to any preceding claim which comprises:
a) a recombinant polynucleotide(s) which encodes polypeptides comprising SEQ ID NO: 1-8 or a variant with at least 95% sequence identity to SEQ ID NO: 1-8; or recombinant polypeptides comprising SEQ ID NO: 1-8 or variants with at least 95% sequence identity to SEQ ID NO: 1-8;
(b) a recombinant polynucleotide(s) which encodes polypeptides comprising SEQ ID NO: 1-3 and 6-8 or variants with at least 95% sequence identity to SEQ ID NO: 1-3 and 6-8; or recombinant polypeptides comprising SEQ ID NO: 1-3 and 6-8 or variants with at least 95% sequence identity to SEQ ID NO: 1-3 and 6-8; or
(c) a recombinant polynucleotide(s) which encode polypeptides comprising SEQ ID NO: 1-5 or variants with at least 95% sequence identity to SEQ ID NO: 1-5; or recombinant polypeptides comprising SEQ ID NO: 1-5 or variants with at least 95% sequence identity to SEQ ID NO: 1-5.

5. A vaccine according to claim 1 wherein the recombinant polynucleotides comprise SEQ ID NO: 9, 10 and 11 or a variant thereof with at least 95% sequence identity.

6. A vaccine according to claim 3 wherein the further polynucleotides comprise one or more of SEQ ID NO: 12-16 or a variant thereof with at least 95% sequence identity.

7. A vaccine according to any preceding claim wherein the polynucleotide is present in a vector.

8. A vaccine according to claim 7 wherein the vector is selected from an adenovirus, a modified vaccinia Ankara vector and a pseudorabies virus vector.

9. A vaccine according to claim 7 or 8 wherein each of the recombinant polynucleotides is provided in the same vector.

10. A vaccine according to any preceding claim for use in treating and/or preventing African swine fever in a subject.

11. A vaccine for use according to claim 10, wherein the subject is a swine subject.

12. A vaccine for use according to claim 11 wherein the subject is a domestic pig.

13. A vaccine for use according to any of claims 10 to 12 wherein the vaccine is administered according to a prime-boost procedure.

14. A vaccine for use according to claim 13 wherein the priming composition comprises one or more adenovirus vectors and the boosting composition comprises one or more modified vaccinia Ankara vectors.

15. A vaccine for use according to any of claims 10 to 14 wherein the vaccine is administered by oral, intravenous, intramuscular, subcutaneous, intranasal or intradermal administration.

## Patentansprüche

1. Impfstoff mit einer Untereinheit des Afrikanische-Schweinepest-Virus (ASFV= African swine fever virus), umfassend:
(i) ein oder mehrere rekombinante Polynukleotide, die Polypeptide codieren, die als Folgendes gezeigt werden:
(a) SEQ ID NO: 1 oder eine Variante mit mindestens 95 % Sequenzidentität dazu,
(b) SEQ ID NO: 2 oder eine Variante mit mindestens 95 % Sequenzidentität dazu, und
(c) SEQ ID NO: 3 oder eine Variante mit mindestens 95 % Sequenzidentität dazu; wobei die Gesamtzahl verschiedener ASFV-Polypeptide, die durch das eine oder die mehreren rekombinanten Polynukleotide codiert werden, 10 oder weniger beträgt; oder
(ii) rekombinante Polypeptide, die als Folgendes gezeigt werden:
(a) SEQ ID NO: 1 oder eine Variante mit mindestens 95 % Sequenzidentität dazu,
(b) SEQ ID NO: 2 oder eine Variante mit mindestens 95 % Sequenzidentität dazu, und
(c) SEQ ID NO: 3 oder eine Variante mit mindestens 95 % Sequenzidentität dazu; wobei der Impfstoff 10 oder weniger verschiedene ASFV-Polypeptide umfasst.

2. Impfstoff nach Anspruch 1, wobei die Anzahl verschiedener ASFV-Polypeptide, die durch das eine oder die mehreren rekombinanten Polynukleotide codiert werden, 9 oder weniger, 8 oder weniger, 7 oder weniger oder 6 oder weniger beträgt; oder wobei der Impfstoff 9 oder weniger, 8 oder weniger, 7 oder weniger oder 6 oder weniger ASFV-Polypeptide umfasst.

3. Impfstoff nach Anspruch 1 oder 2, der Folgendes umfasst:
(i) ein oder mehrere weitere rekombinante Polynukleotide, die ein ASFV-Polypeptid codieren, das aus SEQ ID NO: 4-8 oder einer Variante mit mindestens 95 % Sequenzidentität zu einer aus SEQ ID NO: 4-8 ausgewählt sind; oder
(ii) ein oder mehrere weitere Polypeptide, die aus SEQ ID NO: 4-8 oder einer Variante mit mindestens 95 % Sequenzidentität zu einer aus SEQ ID NO: 4-8 ausgewählt sind.

4. Impfstoff nach einem der vorhergehenden Ansprüche, der Folgendes umfasst:
(a) (ein) rekombinante(s) Polynukleotid(e), das Polypeptide, die SEQ ID NO: 1-8 oder eine Variante mit mindestens 95 % Sequenzidentität zu SEQ ID NO: 1-8 umfassen, oder rekombinante Polypeptide codiert, die SEQ ID NO: 1-8 oder Varianten mit mindestens 95 % Sequenzidentität zu SEQ ID NO: 1-8 umfassen;
(b) (ein) rekombinante (s) Polynukleotid(e), das Polypeptide, die SEQ ID NO: 1-3 und 6-8 oder Varianten mit mindestens 95 % Sequenzidentität zu SEQ ID NO: 1-3 und 6-8 umfassen, oder rekombinante Polypeptide codiert, die SEQ ID NO: 1-3 und 6-8 oder Varianten mit mindestens 95 % Sequenzidentität zu SEQ ID NO: 1-3 und 6-8 umfassen;
(c) (ein) rekombinante(s) Polynukleotid(e), das Polypeptide, die SEQ ID NO: 1-5 oder Varianten mit mindestens 95 % Sequenzidentität zu SEQ ID NO: 1-5 umfassen, oder rekombinante Polypeptide codiert, die SEQ ID NO: 1-5 oder Varianten mit mindestens 95 % Sequenzidentität zu SEQ ID NO: 1-5 umfassen;

5. Impfstoff nach Anspruch 1, wobei die rekombinanten Polynukleotide SEQ ID NO: 9, 10 und 11 oder eine Variante davon mit mindestens 95 % Sequenzidentität umfassen.

6. Impfstoff nach Anspruch 3, wobei die weiteren Polynukleotide eine oder mehrere aus SEQ ID NO: 12-16 oder eine Variante davon mit mindestens 95 % Sequenzidentität umfassen.

7. Impfstoff nach einem vorhergehenden Anspruch, wobei das Polynukleotid in einem Vektor vorliegt.

8. Impfstoff nach Anspruch 7, wobei der Vektor aus einem Adenovirus-, einem Modified-Vaccinia-Ankara-Vektor und einem Pseudorabies-Virus-Vektor ausgewählt ist.

9. Impfstoff nach Anspruch 7 oder 8, wobei die rekombinanten Polynukleotide jeweils in demselben Vektor bereitgestellt werden.

10. Impfstoff nach einem vorhergehenden Anspruch zur Verwendung beim Behandeln und/oder Vorbeugen der Afrikanischen Schweinepest bei einem Individuum.

11. Impfstoff zur Verwendung nach Anspruch 10, wobei es sich bei dem Individuum um ein Schwein handelt.

12. Impfstoff zur Verwendung nach Anspruch 11, wobei es sich bei dem Individuum um ein Hausschwein handelt.

13. Impfstoff zur Verwendung nach einem der Ansprüche 10 bis 12, wobei der Impfstoff gemäß einem Prime-Boost-Verfahren verabreicht wird.

14. Impfstoff zur Verwendung nach Anspruch 13, wobei die Priming-Zusammensetzung einen oder mehrere Adenovirus-Vektoren umfasst und die Boosting-Zusammensetzung einen oder mehrere Modified-Vaccinia-Ankara-Vektoren umfasst.

15. Impfstoff zur Verwendung nach einem der Ansprüche 10 bis 14, wobei der Impfstoff mittels oraler, intravenöser, intramuskulärer, subkutaner, intranasaler oder intradermaler Verabreichung verabreicht wird.

## Revendications

1. Vaccin à sous-unité du virus de la peste porcine africaine (ASFV) comprenant :
(i) un ou plusieurs polynucléotides recombinants qui codent pour des polypeptides présentés comme :
(a) SEQ ID NO: 1 ou un variant ayant au moins 95 % d'identité de séquence avec celle-ci,
(b) SEQ ID NO: 2 ou un variant ayant au moins 95 % d'identité de séquence avec celle-ci, et
(c) SEQ ID NO: 3 ou un variant ayant au moins 95 % d'identité de séquence avec celle-ci ; le nombre total de polypeptides ASFV différents codés par le ou les polynucléotides recombinants étant de 10 ou moins ; ou
(ii) des polypeptides recombinants présentés comme
(a) SEQ ID NO: 1 ou un variant ayant au moins 95 % d'identité de séquence avec celle-ci,
(b) SEQ ID NO: 2 ou un variant ayant au moins 95 % d'identité de séquence avec celle-ci, et
(c) SEQ ID NO: 3 ou un variant ayant au moins 95 % d'identité de séquence avec celle-ci ; le vaccin comprenant 10 polypeptides ASFV différents ou moins.

2. Vaccin selon la revendication 1, dans lequel le nombre de polypeptides ASFV différents codés par le ou les polynucléotides recombinants est de 9 ou moins, 8 ou moins, 7 ou moins, ou 6 ou moins ; ou dans lequel le vaccin comprend 9 ou moins, 8 ou moins, 7 ou moins, ou 6 ou moins polypeptides ASFV.

3. Vaccin selon la revendication 1 ou 2, qui comprend :
(i) un ou plusieurs autres polynucléotides recombinants codant pour un polypeptide ASFV choisi parmi SEQ ID NO: 4-8, ou un variant ayant au moins 95 % d'identité de séquence avec l'une des SEQ ID NO: 4-8 ; ou
(ii) un ou plusieurs polypeptides supplémentaires choisis parmi SEQ ID NO: 4-8 ; ou un variant ayant au moins 95 % d'identité de séquence avec l'une des SEQ ID NO: 4-8.

4. Vaccin selon l'une quelconque des revendications précédentes qui comprend :
(a) un ou plusieurs polynucléotides recombinants qui codent pour des polypeptides comprenant SEQ ID NO: 1-8 ou un variant ayant au moins 95 % d'identité de séquence avec les SEQ ID NO: 1-8 ; ou des polypeptides recombinants comprenant SEQ ID NO: 1-8 ou des variants ayant au moins 95 % d'identité de séquence avec les SEQ ID NO: 1-8 ;
(b) un ou plusieurs polynucléotides recombinants qui codent pour des polypeptides comprenant SEQ ID NO: 1-3 et 6-8 ou des variants ayant au moins 95 % d'identité de séquence avec les SEQ ID NO: 1-3 et 6-8 ; ou des polypeptides recombinants comprenant SEQ ID NO: 1-3 et 6-8 ou des variants ayant au moins 95 % d'identité de séquence avec les SEQ ID NO: 1-3 et 6-8 ; ou
(c) un ou plusieurs polynucléotides recombinants qui codent pour des polypeptides comprenant SEQ ID NO: 1-5 ou des variants ayant au moins 95 % d'identité de séquence avec les SEQ ID NO: 1-5 ; ou des polypeptides recombinants comprenant SEQ ID NO: 1-5 ou des variants ayant au moins 95 % d'identité de séquence avec les SEQ ID NO: 1-5.

5. Vaccin selon la revendication 1, dans lequel les polynucléotides recombinants comprennent SEQ ID NO: 9, 10 et 11 ou un variant de celles-ci ayant au moins 95 % d'identité de séquence.

6. Vaccin selon la revendication 3, dans lequel les autres polynucléotides comprennent un ou plusieurs de SEQ ID NO: 12-16 ou un variant de celle-ci ayant au moins 95 % d'identité de séquence.

7. Vaccin selon l'une quelconque des revendications précédentes, dans lequel le polynucléotide est présent dans un vecteur.

8. Vaccin selon la revendication 7, dans lequel le vecteur est choisi parmi un adénovirus, un vecteur de vaccine Ankara modifié et un vecteur de virus de pseudorage.

9. Vaccin selon la revendication 7 ou 8, dans lequel chacun des polynucléotides recombinants est fourni dans le même vecteur.

10. Vaccin selon l'une quelconque des revendications précédentes destiné à être utilisé dans le traitement et/ou la prévention de la peste porcine africaine chez un sujet.

11. Vaccin destiné à être utilisé selon la revendication 10, dans lequel le sujet est un sujet porcin.

12. Vaccin destiné à être utilisé selon la revendication 11, dans lequel le sujet est un porc domestique.

13. Vaccin destiné à être utilisé selon l'une quelconque des revendications 10 à 12, dans lequel le vaccin est administré selon un protocole de primo-vaccination/rappel.

14. Vaccin destiné à être utilisé selon la revendication 13, dans lequel la composition d'amorçage comprend un ou plusieurs vecteurs adénoviraux et la composition de rappel comprend un ou plusieurs vecteurs de vaccine Ankara modifiés.

15. Vaccin destiné à être utilisé selon l'une quelconque des revendications 10 à 14, dans lequel le vaccin est administré par administration orale, intraveineuse, intramusculaire, sous-cutanée, intranasale ou intradermique.
